# EUROPEAN PATENT APPLICATION

(11) **EP 3 251 585 A1**
(43) Date of publication of application: **06.12.2017**
(21) Application number: 16171999.2
(22) Date of filing: 30.05.2016
(51) Int. Cl.: A61B 5/00, A61M 5/142

(54) **BODY-MOUNTABLE DEVICE**

(71) Applicant: Roche Diabetes Care GmbH, 68305 Mannheim (DE); F. Hoffmann-La Roche AG, 4070 Basel (CH)
(72) Inventor: KRÄMER, Uwe, 68549 Ilvesheim (DE)
(74) Representative: Pfiz, Thomas

(57) **Abstract**

The invention concerns a body-mountable device, in particular a patch for a medical assembly (14), comprising a housing portion (16) adapted to receive the medical assembly (14) and having a bottom side (32), and further comprising a fastening means (18) which is connected to the housing portion (16) and adapted to affix the housing portion (16) to the body (20) of a patient, such that the bottom side (32) is facing the body (20), wherein a fluid-guiding component (22) is configured on the bottom side (32) of the housing portion (16) for guiding a fluid emanating from the body (20) to the periphery of the housing portion (16).

## Description

The invention relates to a body-mountable device, in particular a patch for a medical assembly, according to the preamble of patent claim 1.

Monitoring of certain analytes or agents, specifically glucose or lactate in body fluids like blood or interstitial fluid may be ordinarily performed by so called spot-monitoring on samples extracted from the body or by continuous monitoring via a fully or partially implanted sensor, specifically an electrochemical sensor.

In the latter case the subcutaneously implanted sensor remains in the interstitial tissue over an extended period of time even up to several weeks. Then, the *in vivo* detected measurement signals may be indicative of an analyte, e.g. glucose in the blood of the subject. The monitoring may be a nearly real-time continuous or quasi continuous or periodic approach for frequently providing/updating analyte values without sample handling or similar user interaction.

In present practice, continuous glucose monitoring (CGM)-systems include a so-called bodymount as a patch which comprises a rigid housing portion or mounting platform on which the electronics unit is mounted physically associated with the sensor. However, the bottom side of the housing portion is formed by a largely closed plastic wall which is impermeable for fluid or vapor emanating from the skin area below the housing portion. This can result in fluid pockets forming beneath the housing portion which may cause problems such as skin maceration, skin irritation and plaster wetness, thereby undesirably reducing the possible wearing time.

On this basis the object of the invention is to further improve the known systems and to provide a design with a long-term wear capability.

The combination of features stated in claim 1 is proposed to achieve this object. Advantageous embodiments and further developments of the invention are derived from the dependent claims.

The invention is based on the idea of avoiding the formation of sweat under the housing portion. Accordingly, it is proposed that a fluid-guiding component is configured on the bottom side of the housing portion for guiding a fluid emanating from the body to the periphery of the housing portion. In this way, it is avoided that a flat sealing surface directly rests on the skin, and the fluid or vapor can be discharged under the bottom side of the housing portion to a peripheral site.

Preferably, the fluid-guiding component has a channel-bearing structure with at least one channel therein for the fluid transport. Such a structure, formed as microchannels, may also allow the fluid transport by capillary action.

In another advantageous configuration the fluid-guiding component has a structured uneven surface contour, such that a free space for fluid transport is created.

In this connection it is further advantageous when the fluid-guiding component has at least one of a wave-like, rectangular, trapezoidal or saw-toothed surface contour.

In still another advantageous configuration, the fluid-guiding component comprises a plurality of spacers which are regularly or irregularly distributed over the patch area, such that the clearance between the spacers allows the fluid transport.

A particular embodiment further comprises that the fluid-guiding component is formed as an integral part of the housing portion. This is particularly preferred when the housing portion is placed directly on the skin.

In a particular preferred embodiment, the fastening means is formed as a plaster portion which comprises an adhesive adhering to the skin of the body.

Advantageously, an upper side of the plaster portion and the bottom side of the housing portion are joined via the fluid-guiding component, such that the plaster covers the patch area all over. Alternatively, it is also conceivable that the plaster is only attached over a rim or boundary of the housing portion.

In another advantageous embodiment, the fluid-guiding component is formed by a structured upper surface of the plaster portion.

In order to specifically adapt the fluid transporting ability, the fluid-guiding component may be formed by an additional interface portion placed between the plaster portion and the housing portion.

In an advantageous embodiment, the plaster portion comprises a fluid-transmissible carrier layer, in particular a fabric, such that the fluid is not retained under the plaster.

In order to avoid fluid contamination of the housing interior, the housing portion comprises a wall forming the bottom side, wherein the wall is impermeable for the fluid.

Preferably, the patch is designed for the transportation of fluid or sweat that is formed by perspiration.

It is also possible to avoid the formation of sweat in cases where the fastening means is formed by one of a band, strap or belt, in particular a wristband or a chest strap or hip belt.

In preferred embodiments the body-mountable device is an instrument for analyte monitoring, in particular continuous glucose monitoring or an insulin pump or a blood pressure monitor.

In the following, the invention is further elucidated on the basis of embodiment examples shown schematically in the drawings, where
- Fig. 1: is a sectional view of a body-mountable patch for a medical sensor assembly including a fluid-permeable interface;
- Fig. 2 to 4: illustrate various embodiments of the fluid-permeable interface;
- Fig. 5: is a perspective view of a profiled fluid-permeable interface;
- Fig. 6: is a perspective view of a fluid-permeable interface provided by discrete spacers.

Referring to the drawings, a system 10 for continuous analyte monitoring in a body fluid, specifically continuous glucose monitoring, comprises a body-mountable patch 12 and a medical assembly 14, wherein the patch 12 includes a housing portion 16 providing a receiving space 17 for the sensor assembly 14 and a plaster portion 18 for adhering the patch 12 to the body 20 of a patient, and wherein the housing portion 16 and the plaster portion 18 are connected via a fluid-guiding component 22.

The sensor assembly 14 may include a subcutaneously insertable analyte sensor and/or measurement electronics and/or an infusion set, e.g. as further detailed in EP 1 971 256 B1.

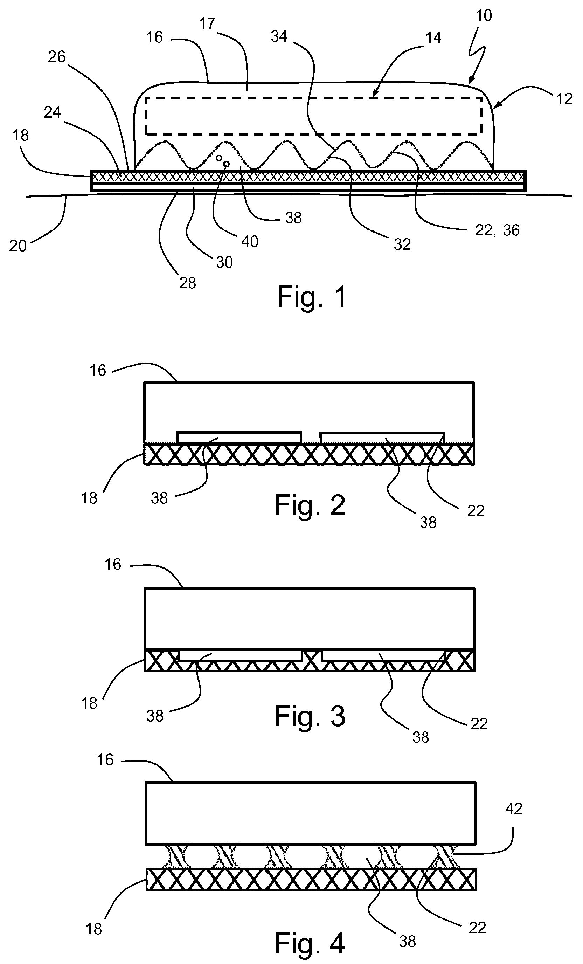

As further detailed in Fig. 1, the plaster portion 18 has a sweat-permeable fabric 24 as a carrier layer, which has a free upper side 26 facing away from the body 20, while the lower side 28 comprises an adhesive 30, in particular a pressure sensitive adhesive for adhesive attachment on the skin of the body 20.

The housing portion 16 may be formed as a molded part from a plastic material. On its bottom side 32, the plastic material forms a wall 34 which is generally blocking the transport of sweat in a direction perpendicular to the skin of the body 20.

In order to allow the fluid formed by perspiration to escape laterally out of the housing area, the fluid-guiding component 22 has a channel-bearing structure 36 with at least one channel 38 therein. Thus, the sweat 40 which permeates the plaster portion 18 is guided or transported in the channel 38 in a direction away from the housing portion 16. For this purpose, the embodiment in Fig. 1 comprises a wave-like structured surface contour.

As also apparent from Fig. 2, the fluid-guiding component 22 can be formed as an integral part of the housing portion 16. Alternatively, the fluid-guiding component 22 may be provided as a structured upper surface of the plaster portion 18. Still another embodiment comprises a fluid-guiding component 22 that is formed by an additional interface portion 42 placed between the plaster portion 18 and the housing portion 16, as illustrated in Fig. 4.

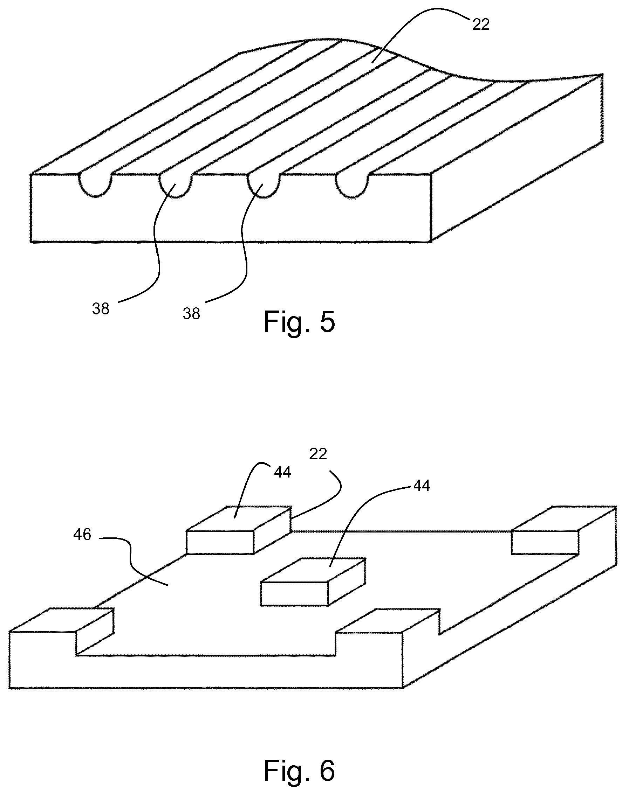

Fig. 5 shows a fluid-guiding component 22 designed as a profiled channel structure, such that the individual channels are aligned parallel to each other.

In the embodiment of Fig. 6, the fluid-guiding component 22 comprises a plurality of spacers 44 which are regularly or irregularly distributed over the area of the plaster or housing portion, such that the clearance 44 between the spacers 466 allows a multidirectional fluid transport to the circumference or periphery of the housing portion 16.

## Claims

1. Body-mountable device, in particular patch for a medical assembly (14), comprising:
a) a housing portion (16) providing a receiving space (17) and having a bottom side (32),
b) a fastening means (18) which is connected or connectable to the housing portion (16) and is adapted to affix the housing portion (16) to the body (20) of a patient, such that the bottom side (32) is facing the body (20),
c) **characterized in that** a fluid-guiding component (22) is configured on the bottom side (32) of the housing portion (16) for guiding a fluid emanating from the body (20) to the periphery of the housing portion (16).

2. The device of claim 1, wherein the fluid-guiding component (22) has a channel-bearing structure with at least one channel (38) therein for the fluid transport.

3. The device of claim 1 or 2, wherein the fluid-guiding component (22) has a structured uneven surface contour.

4. The device according to any of claims 1 to 3, wherein the fluid-guiding component (22) has at least one of a wave-like, rectangular, trapezoidal or saw-toothed surface contour.

5. The device according to any of claims 1 to 4, wherein the fluid-guiding component (22) comprises a plurality of spacers (44) which are regularly or irregularly distributed over the device area, such that the clearance (46) between the spacers (44) allows the fluid transport.

6. The device according to any of claims 1 to 5, wherein the fluid-guiding component (22) is formed as an integral part of the housing portion (16).

7. The device according to any of claims 1 to 6, wherein the fastening means (18) is formed as a plaster portion which comprises an adhesive (30) adhering to the skin of the body (20).

8. The device of claim 7, wherein an upper side (26) of the plaster portion (18) and the bottom side (32) of the housing portion (16) are joined via the fluid-guiding component (22).

9. The device of claim 7 or 8, wherein the fluid-guiding component (22) is formed by a structured upper surface of the plaster portion (18).

10. The device according to any of claims 7 to 9, wherein the fluid-guiding component (22) is formed by an additional interface portion (42) placed between the plaster portion (18) and the housing portion (16).

11. The device according to any of claims 7 to 10,
wherein the plaster portion (18) comprises a fluid-transmissible carrier layer (24), in particular a fabric.

12. The device according to any of claims 1 to 11,
wherein the housing portion (16) comprises a wall (34) forming the bottom side (32), wherein the wall (34) is impermeable for the fluid.

13. The device according to any of claims 1 to 12,
wherein the fluid is formed by perspiration.

14. The device according to any of claims 1 to 13,
wherein the fastening means (18) is formed by one of a band, strap or belt, in particular a wristband or a chest strap or hip belt.

15. The device according to any of claims 1 to 14,
wherein the medical assembly (14) is an instrument for analyte monitoring, in particular continuous glucose monitoring or an insulin pump or a blood pressure monitor.
